# EUROPEAN PATENT APPLICATION

(11) **EP 2 022 500 A1**
(43) Date of publication of application: **11.02.2009**
(21) Application number: 06747079.9
(22) Date of filing: 01.06.2006
(51) Int. Cl.: A61K 31/505, A61K 31/421, A61K 31/423, A61K 31/428, A61P 1/00, A61P 35/00, A61P 35/04, C07D 239/34, C07D 263/32, C07D 263/56, C07D 277/66

(54) **TUMOR SUPPRESSOR**

(71) Applicant: JAPAN as represented by DIRECTOR GENERAL OF AGENCY OF NATIONAL CANCER CENTER, Chuo-ku, Tokyo 104-0045 (JP); Shizuoka Coffein Co., Ltd., Shizuoka-shi, Shizuoka 420-0008 (JP)
(72) Inventor: WAKABAYASHI, Keiji, Tokyo 146-0092 (JP); MUTOH, Michihiro, Tokyo 157-0074 (JP); NAKATOGAWA, Kiyoshi, Shizuoka-shi Shizuoka 420-0886 (JP); TAKAGI, Masamichi, Shizuoka-shi Shizuoka 421-0101 (JP); AKASHIMA, Makoto, Shizuoka-shi Shizuoka 422-8007 (JP); UEDA, Kentaro, Shizuoka-shi Shizuoka 420-0007 (JP)
(74) Representative: Duckworth, Timothy John
(86) International application number: PCT/JP2006/310977
(87) International publication number: WO 2007/138705

(57) **Abstract**

A compound having a sructure of propanedioic acid represented by the following formula (1): 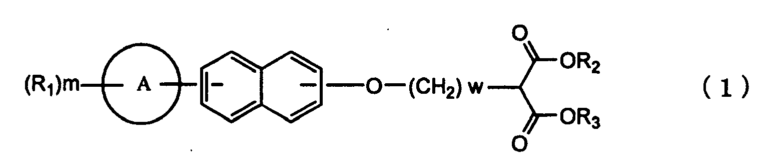 (1) (wherein the ring A represents oxazole or the like; R₁ represents an alkyl group, a lower alkoxy group or the like; R₂ and R₃ represent a lower alkyl group or the like; m represents 0 or an integer of 1 to 4, and m pieces of R₁ may be the same or different from each other; and W represents an integer of 3 to 5) is useful as an agent for preventing or treating a gastrointestinal polyp and/or a malignant tumor, or an agent for preventing metastasis thereof.

## Description

### TECHNICAL FIELD

The present invention relates to an agent for preventing or treating gastrointestinal polyp and/or malignant tumor, or an agent for preventing the metastasis of malignant tumor, which comprises a compound having a propanedioic acid structure as an active ingredient.

### BACKGROUND ART

Therapeutic methods such as chemotherapy, surgical treatment, radiation treatment, thermotherapy, immunotherapy and photodynamic therapy are used in the treatment of malignant tumor. In recent years, in the field of chemotherapy, as part of search for an anti-malignant tumor agent with few side effects, various types of medicaments having action mechanisms different from those of the conventional anti-malignant tumor agents have been developed. For example, the development of a molecular-targeted agent that targets for molecules specifically expressed in malignant tumor has been progressed. To date, a therapeutic agent for non-small-cell lung cancer and a therapeutic agent for chronic myelocytic leukemia have been developed (Patent Documents 1 and 2).
In addition, in studies of a therapeutic agent for large intestinal cancer using a cyclooxygenase-2 (COX-2) selective inhibitor, it has been reported that such therapeutic agent suppresses the intestinal cancer of a test animal (Non-Patent Document 1). These agents have been anticipated as chemotherapeutic agents with few side effects.
However, the use of such chemotherapeutic agents has often been limited because of their harmful effects due to side effects, such as the onset of interstitial pneumonia due to the side effects of a molecular-targeted agent for treating non-small-cell lung cancer or the occurrence of cardiovascular risk due to administration of a COX-2 inhibitor. Thus, it has been desired to develop an anti-malignant tumor agent with high safety and efficacy.

In particular, with regard to the aforementioned large intestinal cancer, recently, the morbidity rate of large intestinal cancer has continuously increased in Japan. The establishment of a preventive measure against large intestinal cancer has become an important issue. Similarly, lifestyle related diseases such as adiposis, diabetes or hyperlipidemia have become social issues. The relationship between such adiposis, diabetes or hyperlipidemia (hypercholesterolemia or hypertriglyceridemia) and large intestinal cancer has been suggested in both epidemiologic study and experimental carcinogenesis using test animals. However, the mechanism thereof has not yet been clarified so far.
In recent years, it has been reported that adipocytokine, a physiologically active substance mainly released from enlarged fat cells, is deeply involved not only in diabetes or metabolic syndrome, but also in carcinogenesis. Such adipocytokines reportedly involved in carcinogenesis include plasminogen activator inhibitor-1 (PAI-1), adiponectin, TNF, and leptin.

With regard to the relationship between carcinogenesis and PAI-1, for example, Non-Patent Document 2 describes that expression of PAI-1 was increased in the polyp of a patient with familial adenomatous polyposis, and that when PAI-1 was subjected to homo-deletion in an Apc1638N mouse, a model mouse of familial adenomatous polyposis, development of polyps in small intestine was decreased. Non-Patent Document 3 describes high expression of PAI-1 in human ovarian cancer. Non-Patent Document 4 describes the positive correlation of expression of PAI-1 in the atypical epithelium of human stomach with the stage of progressive stomach cancer. Non-Patent Document 5 describes survivin (an apoptosis inhibitor) in 420 human breast cancer patients positively correlated with high expression of PAI-1. Non-Patent Document 6 describes that PAI-1 is involved in infiltration of oral squamous cell carcinoma. Non-Patent Document 7 describes that, in studies regarding the survival rate of 99 pulmonary adenomatosis patients, the low survival rate positively correlated with expression of PAI-1. Non-Patent Document 8 describes that PAI-1 promotes the migration of fibrosarcoma cells. Non-patent Document 9 describes that the severity of a human breast cancer patient correlates with the 4G/5G polymorphism of PAI-1. Non-Patent Document 10 describes that PAI-1 promotes proliferation of fibrosarcoma cells.

Moreover, with regard to the use of a PAI-1-inhibiting compound as an antitumor agent, for example, Patent Document 3 discloses that administration of a substituted indole oxo-acetyl amino acetic acid derivative as an inhibiting agent for plasminogen activator inhibitor-1 (PAI-1) is useful as a method for treating cancer.
On the other hand, Patent Documents 4, 5 and 6 disclose propanedioic acid derivatives having PAI-1-inhibiting action. However, in Patent Documents 4, 5 and 6, such PAI-1-inhibiting compounds are used as thrombolytic agents or antithrombotic agents. The effectiveness of such propanedioic acid derivatives as agents for preventing or treating malignant tumor, or as agents for preventing the metastasis of malignant tumor, has not yet been studied.
[Patent Document 1] JP-A-10-508616
[Patent Document 2] JP-A-6-87834
[Patent Document 3] JP-A-2006-510672
[Patent Document 4] International Publication WO04/011442 pamphlet
[Patent Document 5] International Publication WO04/10996 pamphlet
[Patent Document 6] JP-A-2004-250401
[Non-Patent Document 1] Cancer Research., 58, 409-412 (1998)
[Non-Patent Document 2] Oncogene., 24: 1615-1624.(2005)
[Non-Patent Document 3] Anticancer Res., 26(2C): 1683-1689 (2006)
[Non-Patent Document 4] Cancer., 106:1026-1035 (2006)
[Non-Patent Document 5] Ann Oncol., 17:597-604 (2006)
[Non-Patent Document 6] Oncol Rep., 15:393-400 (2006)
[Non-Patent Document 7] Lung Cancer., 51:193-200 (2006)
[Non-Patent Document 8] Semin Thromb Hemost., 31:356-63 (2005)
[Non-Patent Document 9] Thromb Res., 117:487-492 (2006)
[Non-Patent Document 10] Cancer Res., 15; 60: 5839-5847 (2000)

### DISCLOSURE OF THE INVENTION

### PROBLEMS TO BE SOLVED BY THE INVENTION

It is an object of the present invention to provide an agent for preventing or treating gastrointestinal polyp and/or malignant tumor, or an agent for preventing the metastasis of malignant tumor, which uses a propanedioic acid derivative having PAI-1-inhibiting action.

### MEANS FOR SOLVING THE PROBLEMS

The present inventors have focused on the relationship between PAI-1 and the development of gastrointestinal polyp or malignant tumor. The inventors have conducted intensive studies directed towards developing a novel agent for preventing or treating gastrointestinal polyp and/or malignant tumor, or a novel agent for preventing the metastasis of malignant tumor, which comprises a PAI-1-inhibiting compound having a propanedioic acid structure, thereby completing the present invention.

That is to say, the present invention relates to an agent for preventing or treating gastrointestinal polyp and/or malignant tumor, or an agent for preventing the metastasis of malignant tumor, which comprises a propanedioic acid derivative represented by the following formula (1), a nontoxic salt thereof, a solvate thereof, or a prodrug thereof, as an active ingredient, 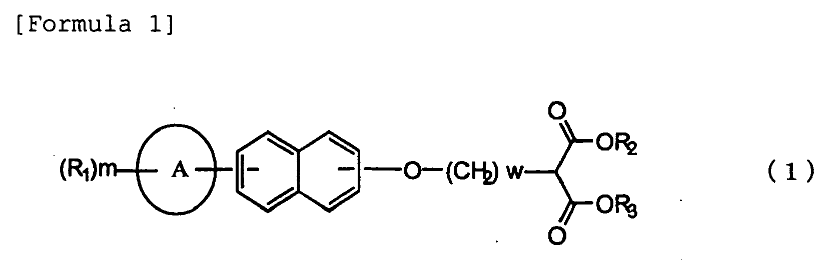 wherein ring A represents oxazole, benzoxazole, benzothiazole, or pyrimidine; R₁ represents a hydroxyl group, an alkyl group, a lower alkoxy group, a cyclohexylmethoxy group, a benzyloxy group (wherein the phenyl of the benzyloxy group may be substituted with a substituent selected from among a lower alkyl group, a lower alkoxy group, and a halogen atom), a trifluoromethyl group, a nitro group, a -N(R₄,R₅) group (wherein each of R₄ and R₅ independently represents a hydrogen atom, an alkyl group, or a benzyl group), a halogen atom, or a phenyl group (wherein the phenyl group may be substituted with a substituent selected from among a lower alkyl group, a lower alkoxy group, and a halogen atom); each of R₂ and R₃ independently represents a hydrogen atom or a lower alkyl group; m represents 0 or an integer of 1 to 4, and an m number of R₁ may be identical to or different from one another; and W represents an integer of 3 to 5.
Moreover, the present invention relates to use of the propanedioic acid derivative represented by formula (1), a nontoxic salt thereof, a solvate thereof, or a prodrug thereof for production of an agent for preventing or treating gastrointestinal polyp and/or malignant tumor, or an agent for preventing the metastasis of malignant tumor.
Furthermore, the present invention relates to a method for preventing or treating gastrointestinal polyp and/or malignant tumor, or preventing the metastasis of malignant tumor, which comprises administration of the propanedioic acid derivative represented by formula (1), a nontoxic salt thereof, a solvate thereof, or a prodrug thereof to humans or animals.

### ADVANTAGES OF THE INVENTION

The propanedioic acid derivative of the present invention significantly suppressed the development of gastrointestinal polyp and malignant tumor in an *in vivo* test. Accordingly, the compound of the present invention can be used as an agent for preventing or treating gastrointestinal polyp, gastrointestinal cancer (for example, esophageal cancer, stomach cancer, duodenal cancer, small intestinal cancer, large intestinal cancer (e.g. colon cancer, rectal cancer), etc.), lung cancer, breast cancer, pancreatic cancer, liver cancer, uterine cancer, ovarian cancer, epithelial malignant tumor, prostatic cancer, leukemia, malignant lymphoma, multiple myeloma, and the like, and/or as an agent for preventing the metastasis of such malignant tumor. In particular, the propanedioic acid derivative of the present invention is useful as an agent for preventing or treating gastrointestinal polyp and gastrointestinal cancer (for example, esophageal cancer, stomach cancer, duodenal cancer, small intestinal cancer, large intestinal cancer (e.g. colon cancer, rectal cancer), etc.), and/or as an agent for preventing the metastasis of such malignant tumor.

### BEST MODE FOR CARRYING OUT THE INVENTION

In the present invention, the term "alkyl group" is used to mean a linear or branched alkyl group containing 1 to 6 carbon atoms. Specific examples of such an alkyl group include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, an isohexyl group, and a neohexyl group. The term "lower alkyl group" is used to mean a linear or branched alkyl group containing 1 to 4 carbon atoms. Specific examples of such a lower alkyl group include a methyl group, an ethyl group, a propyl group, an isopropyl group, a butyl group, an isobutyl group, a sec-butyl group, and a tert-butyl group.
The term "lower alkoxy group" is used to mean a linear or branched alkoxy group containing 1 to 4 carbon atoms. Specific examples of such a lower alkoxy group include a methoxy group, an ethoxy group, a propoxy group, an isopropoxy group, a butoxy group, an isobutoxy group, a sec-butoxy group, and a tert-butoxy group. A methoxy group and an ethoxy group are preferable.
The term "halogen atom" specifically includes a chlorine atom, a bromine atom, and a fluorine atom.

In the propanedioic acid derivative represented by formula (1) of the present invention, ring A represents oxazole, benzoxazole, benzothiazole, or pyrimidine. When such ring A represents oxazole, a propanedioic acid derivative represented by the following formula (2) is preferable: 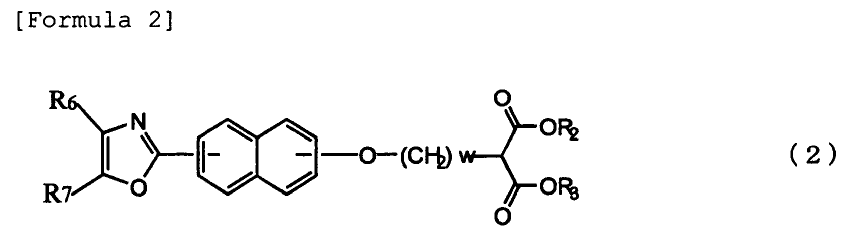 wherein R₂, R₃, and W have the same meanings as those described above, and R₆ and R₇ identically or differently represent a hydrogen atom, an alkyl group, or a phenyl group (wherein the phenyl group may be substituted with a substituent selected from among a lower alkyl group, a lower alkoxy group, and a halogen atom). Specific preferred examples of such a propanedioic acid derivative include the following compounds:
[5-[[6-(2-oxazolyl)-2-naphthalenyl]oxy]pentyl]propanedioic acid;
[5-[[6-(4,5-dimethyl-2-oxazolyl)-2-naphthalenyl]oxy]pentyl]propanedioic acid diethyl ester;
[5-[[6-[4-(1,1-dimethylethyl)-2-oxazolyl]-2-naphthalenyl]oxy]pentyl]propanedioic acid;
[5-[[6-(5-phenyl-2-oxazolyl)-2-naphthalenyl]oxy]pentyl]propanedioic acid diethyl ester;
[5-[[6-[4-(4-chlorophenyl)-2-oxazolyl]-2-naphthalenyl]oxy]pentyl]propanedioic acid diethyl ester;
[5-[[6-(4,5-diphenyl-2-oxazolyl)-2-naphthalenyl]oxy]pentyl]propanedioic acid;
[5-[[6-[4,5-bis(4-methylphenyl)-2-oxazolyl]-2-naphthalenyl]oxy]pentyl]propanedioic acid;
[5-[[6-[4,5-bis(4-methoxyphenyl)-2-oxazolyl]-2-naphthalenyl]oxy]pentyl]propanedioic acid diethyl ester; and
[5-[[6-[4,5-bis(4-methoxyphenyl)-2-oxazolyl]-2-naphthalenyl]oxy]pentyl]propanedioic acid.

In the propanedioic acid derivative represented by formula (1) of the present invention, when ring A represents benzoxazole or benzothiazole, a propanedioic acid derivative represented by the following formula (3) is preferable: 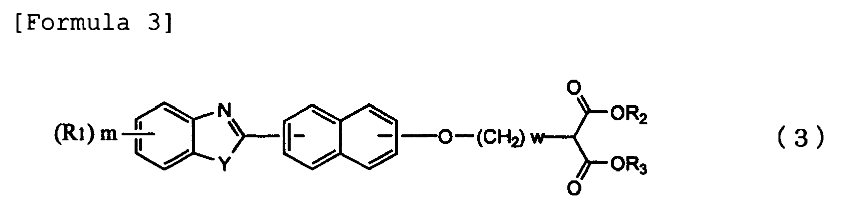 wherein Y represents an oxygen atom or a sulfur atom, and R₁, R₂, R₃, m, and W have the same meanings as those described above. Among such propanedioic acid derivatives, when ring A represents benzoxazole, a propanedioic acid derivative represented by the following formula (4) is more preferable: 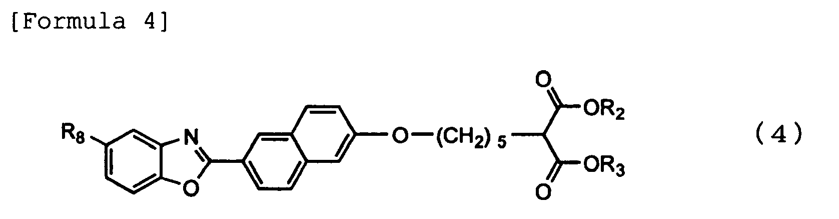 wherein R₂ and R₃ have the same meanings as those described above, and R₈ represents an alkyl group. Among others, a propanedioic acid derivative represented by the following formula (5) is particularly preferable: 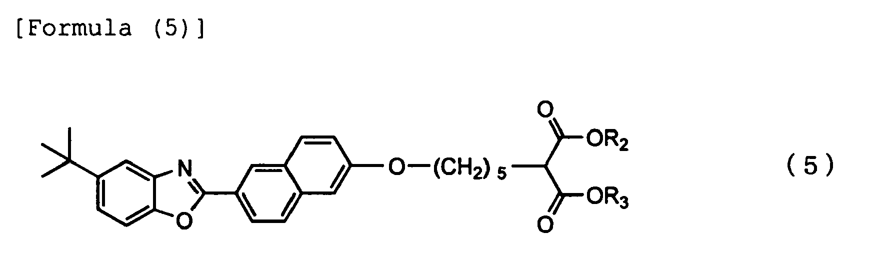 wherein R₂ and R₃ have the same meanings as those described above.
Specific preferred examples of such a propanedioic acid derivative, when ring A is benzoxazole, include the following compounds:
[3-[[6-(2-benzoxazolyl)-2-naphthalenyl]oxy]propyl]propanedioic acid;
[3-[[6-(4-methyl-2-benzoxazolyl)-2-naphthalenyl]oxy]propyl]propanedioic acid;
[3-[[6-(5-phenyl-2-benzoxazolyl)-2-naphthalenyl]oxy]propyl]propanedioic acid diethyl ester;
[3-[[6-(5-phenyl-2-benzoxazolyl)-2-naphthalenyl]oxy]propyl]propanedioic acid;
[3-[[6-(5-chloro-2-benzoxazolyl)-2-naphthalenyl]oxy]propyl]propanedioic acid;
[3-[[3-(5-chloro-2-benzoxazolyl)-2-naphthalenyl]oxy]propyl]propanedioic acid;
[3-[[5-[5-(1,1-dimethylethyl)-2-benzoxazolyl]-2-naphthalenyl]oxy]propyl]propanedioic acid;
[3-[[5-(5-chloro-2-benzoxazolyl)-2-naphthalenyl]oxy]propyl]propanedioic acid diethyl ester;
[5-[[6-(4-methyl-2-benzoxazolyl)-2-naphthalenyl]oxy]pentyl]propanedioic acid;
[5-[[6-(6-methyl-2-benzoxazolyl)-2-naphthalenyl]oxy]pentyl]propanedioic acid;
[5-[[6-[5-(1,1-dimethylethyl)-2-benzoxazolyl]-2-naphthalenyl]oxy]pentyl]propanedioic acid;
[5-[[6-(6-methoxy-2-benzoxazolyl)-2-naphthalenyl]oxy]pentyl]propanedioic acid diethyl ester;
[5-[[6-(6-methoxy-2-benzoxazolyl)-2-naphthalenyl]oxy]pentyl]propanedioic acid;
[5-[[6-[6-(cyclohexylmethoxy)-2-benzoxazolyl]-2-naphthalenyl]oxy]pentyl]propanedioic acid;
[5-[[6-[6-(phenylmethoxy)-2-benzoxazolyl]-2-naphthalenyl]oxy]pentyl]propanedioic acid;
[5-[[6-(5-chloro-2-benzoxazolyl)-2-naphthalenyl]oxy]pentyl]propanedioic acid;
[5-[[6-(6-nitro-2-benzoxazolyl)-2-naphthalenyl]oxy]pentyl]propanedioic acid diethyl ester;
[5-[[6-[6-(diethylamino)-2-benzoxazolyl]-2-naphthalenyl]oxy]pentyl]propanedioic acid disodium salt;
[5-[[3-(6-methyl-2-benzoxazolyl)-2-naphthalenyl]oxy]pentyl]propanedioic acid;
[5-[[3-[5-(1,1-dimethylethyl)-2-benzoxazolyl]-2-naphthalenyl]oxy]pentyl]propanedioic acid;
[5-[[3-(5-phenyl-2-benzoxazolyl)-2-naphthalenyl]oxy]pentyl]propanedioic acid;
[5-[[3-(5-chloro-2-benzoxazolyl)-2-naphthalenyl]oxy]pentyl]propanedioic acid;
[5-[[3-(6-nitro-2-benzoxazolyl)-2-naphthalenyl]oxy]pentyl]propanedioic acid diethyl ester;
[5-[[5-(6-methyl-2-benzoxazolyl)-2-naphthalenyl]oxy]pentyl]propanedioic acid;
[5-[[5-[5-(1,1-dimethylethyl)-2-benzoxazolyl]-2-naphthalenyl]oxy]pentyl]propanedioic acid;
[5-[[5-[6-(cyclohexylmethoxy)-2-benzoxazolyl]-2-naphthalenyl]oxy]pentyl]propanedioic acid diethyl ester;
[5-[[5-(5-phenyl-2-benzoxazolyl)-2-naphthalenyl]oxy]pentyl]propanedioic acid;
[5-[[5-(5-chloro-2-benzoxazolyl)-2-naphthalenyl]oxy]pentyl]propanedioic acid diethyl ester;
[5-[[5-(6-nitro-2-benzoxazolyl)-2-naphthalenyl]oxy]pentyl]propanedioic acid diethyl ester; and
[5-[[5-[6-(diethylamino)-2-benzoxazolyl]-2-naphthalenyl]oxy]pentyl]propanedioic acid.

Specific preferred examples of a propanedioic acid derivative wherein, in formula (3), ring A is benzothiazole include the following compounds:
[3-[[6-(2-benzothiazolyl)-2-naphthalenyl]oxy]propyl]propanedioic acid; and
[5-[[6-(2-benzothiazolyl)-2-naphthalenyl]oxy]pentyl]propanedioic acid diethyl ester.

In the propanedioic acid derivative represented by formula (1) of the present invention, when ring A represents pyrimidine, a propanedioic acid derivative represented by the following formula (6) is preferable: 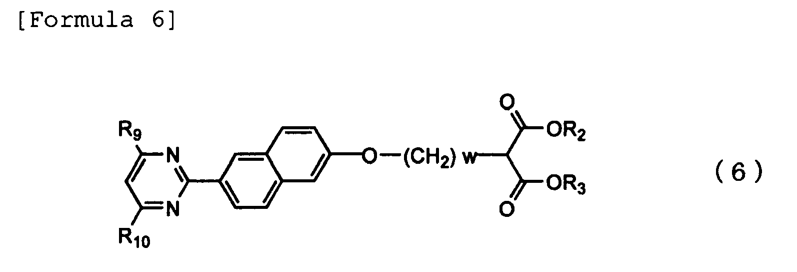 wherein R₂, R₃, and W have the same meanings as those described above; R₉ represents a hydroxyl group, a cyclohexylmethoxy group, or a benzyloxy group (wherein the phenyl of the benzyloxy group may be substituted with a substituent selected from among a lower alkyl group, a lower alkoxy group, and a halogen atom); and R₁₀ represents an alkyl group, a trifluoromethyl group, or a phenyl group (wherein the phenyl group may be substituted with a substituent selected from among a lower alkyl group, a lower alkoxy group, and a halogen atom). Specific preferred examples of such a propanedioic acid derivative include the following compounds:
[3-[[6-[4-[(2-fluorophenyl)methoxy]-6-phenyl-2-pyrimidinyl]-2-naphthalenyl]oxy]propyl]propanedioic acid;
[3-[[6-[4-[(2-fluorophenyl)methoxy]-6-(4-methoxyphenyl)-2-pyrimidinyl]-2-naphthalenyl]oxy]propyl]propanedioic acid;
[5-[[6-[1,4-dihydro-4-oxo-6-(trifluoromethyl)-2-pyrimidinyl]-2-naphthalenyl]oxy]pentyl]propanedioic acid;
[5-[[6-(1,4-dihydro-4-oxo-6-phenyl-2-pyrimidinyl)-2-naphthalenyl]oxy]pentyl]propanedioic acid;
[5-[[6-[4-phenyl-6-(phenylmethoxy)-2-pyrimidinyl]-2-naphthalenyl]oxy]pentyl]propanedioic acid ethyl ester;
[5-[[6-[4-phenyl-6-(phenylmethoxy)-2-pyrimidinyl]-2-naphthalenyl]oxy]pentyl]propanedioic acid;
[5-[[6-[4-cyclohexylmethoxy-6-(1,1-dimethylethyl)-2-pyrimidinyl]-2-naphthalenyl]oxy]pentyl]propanedioic acid;
[5-[[6-[4-(1,1-dimethylethyl)-6-[(4-fluorophenyl)methoxy]-2-pyrimidinyl]-2-naphthalenyl]oxy]pentyl]propanedioic acid; and
[5-[[6-[4-[(2-chlorophenyl)methoxy]-6-(1,1-dimethylethyl)-2-pyrimidinyl]-2-naphthalenyl]oxy]pentyl]propanedioic acid.

The propanedioic acid derivative of the present invention may also be a nontoxic salt thereof, a solvate thereof, or a prodrug thereof. The nontoxic salt includes a pharmacologically acceptable metal salt, organic amine salt, and acid-added salt. Specifically, the metal salt includes, for example, an alkaline metal salt such as a sodium salt or a potassium salt, and an alkaline-earth metal salt such as a magnesium salt or a calcium salt. The acid-added salt includes an inorganic acid salt such as a hydrochloride, a phosphate or a sulfate, and an organic acid salt such as a methanesulfonate. The solvate includes a hydrate. The prodrug includes a compound that is converted to the propanedioic acid derivative of formula (1) by the action of a gastric acid, an enzyme, etc. in vivo. Examples of the prodrug of the propanedioic acid derivative of formula (1) include: a prodrug obtained by binding a carboxylic acid containing 2 to 8 carbon atoms or an alcohol compound containing 1 to 7 carbon atoms to a hydroxyl group or a carboxyl group existing in the molecule of the propanedioic acid derivative of formula (1) via an ester bond; and a prodrug obtained by binding an amino group existing in the molecule of the propanedioic acid derivative of formula (1) to a carboxylic acid containing 2 to 8 carbon atoms via an amide bond.

The propanedioic acid derivatives represented by formulae (1) to (6) used in the present invention are all known compounds. These compounds can be produced by the methods described in International Publication WO04/011442 pamphlet, International Publication WO04/10996 pamphlet, JP-A-2004-250401, JP-A-2004-011442, JP-A-2004-010996, JP-A-2005-320346, etc.

The present inventors have carried out a carcinogenesis suppression test on the propanedioic acid derivatives represented by formulae (1) to (6), which had been known as PAI-1-inhibiting compounds.
That is to say, the inventors used, as a test system, familial adenomatous polyposis (FAP) model mice (Apc gene hetero-deficient mice) belonging to a high risk group of large intestinal cancer. These model mice have two characteristics. That is, the serum triglyceride value of this type of mouse is sharply increased as the mouse gets older, and a large number of polyps are developed by activation of a Wnt signal. Thus, this mouse can be used as an experimental animal model useful for examining the relationship between a hyperlipidemia condition and large intestinal cancer. In this test, based on the fact that expression of PAI-1 was increased in the polyps of FAP patients and that expression of PAI-1 was induced by triglyceride, the action of suppressing the development of intestinal polyps of a PAI-1-inhibiting compound was evaluated using Min mice that were one type of Apc gene-deficient mice. By carrying out such a test system, the inventors discovered that the propanedioic acid derivatives represented by formulae (1) to (6) of the present invention significantly suppressed the development of gastrointestinal polyp and malignant tumor, and thus that they were extremely effective as agents for preventing or treating gastrointestinal polyp and/or malignant tumor, or as agents for preventing the metastasis of malignant tumor.

A pharmaceutical preparation comprising, as an active ingredient, the propanedioic acid derivative represented by formulae (1) to (6) can be generally administered to mammals (including human patients) in the form of an oral preparation such as a tablet, a capsule, a powder, a granule or a syrup, a rectal preparation, or an injection. In addition, the propanedioic acid derivative of the present invention can be administered in the form of a single therapeutic agent or a mixture with other therapeutic agents.
Such a propanedioic acid derivative may be administered singly, but in general, it is administered in the form of a pharmaceutical composition. Such a pharmaceutical preparation can be produced by adding pharmacologically and pharmaceutically acceptable additives to the propanedioic acid derivative and then applying an ordinary method. Namely, for production of an oral preparation, commonly used additives such as an excipient, a lubricant, a binder, a disintegrator, a wetting agent or a coating agent can be used. Such an oral liquid preparation may be in the form of an aqueous or an oily suspension, a solution, an emulsion, a syrup, or an elixir. Otherwise, it may also be provided in the form of a dry syrup that is prepared with water or other suitable solvent before use. The aforementioned liquid agent may comprise common additives such as a suspending agent, a perfume, a diluent or an emulsifier. When the pharmaceutical preparation is administered via an intrarectal administration, it can be administered in the form of a suppository. Such a suppository can be produced by using, as a base, a suitable substance such as cocoa butter, laurin butter, macrogol, glycerinated gelatin, Witepsol, sodium stearate or a mixture thereof, and as necessary, adding an emulsifier, a suspending agent, a preservative or the like to the base. For an injection, pharmaceutical components which are able to form an aqueous solution or a dosage form that is dissolved when used, such as a distilled water, a normal saline solution, a 5% glucose solution, a solubilizer or solubilizing agent such as propylene glycol, a pH adjuster, a isotonizing agent, or a stabilizer are used. Specific examples of the excipient and other additives used in the aforementioned composition are given below.

Examples of the excipient include magnesium aluminometasilicate, magnesium silicate, magnesium carbonate, calcium hydrogen phosphate, Avicel, various types of starches, dextrin, carboxymethyl starch (CMS), and lactose. Examples of the binder include ethyl cellulose (EC), hydroxypropylmethylcellulose (HPMC), hydroxypropylcellulose (HPC), sodium alginate, gelatin, and polyvinyl pyrrolidone (PVP). Examples of the disintegrator include synthetic aluminum silicate, magnesium aluminometasilicate, Avicel, and hydroxypropyl starch (CPS). Examples of the anticaking agent include light anhydrous silicic acid and synthetic aluminum silicate. Examples of the lubricant include synthetic aluminum silicate, silicic acid anhydride, talc, and Avicel. Examples of the corrective include mannitol, citric acid, Na citrate, and sugar. Examples of the emulsifier include gelatin, macrogol (PEG), propylene glycol fatty acid ester, polyoxyethylene polyoxypropylene glycol, and phospholipid. Examples of the stabilizer include propylene glycol fatty acid ester, polyoxyethylene polyoxypropylene glycol, and various types of natural or synthetic cyclodextrins. Examples of the absorbefacient include propylene glycol fatty acid ester, polyoxyethylene polyoxypropylene glycol, propylene glycol, sodium lauryl sulfate, and various types of natural or synthetic cyclodextrins. Examples of the solubilizer include ethanol, polyethylene glycol, propylene glycol fatty acid ester, propylene glycol, and various types of natural or synthetic cyclodextrins. Examples of the suspending agent include sodium alginate, gelatin, propylene glycol, and sodium lauryl sulfate. Examples of the coating agent include magnesium silicate, talc, titanium oxide, calcium carbonate, triacetin, and carboxy methyl ethyl cellulose (CMEC). Examples of the coloring agent include tar color and caramel.

When the propanedioic acid derivative of the present invention is administered to a human, the dose is different depending on the age or condition of the patient, etc. In the case of an adult, in general, approximately 1 to 1,000 mg/adult/day of an oral preparation or a rectal preparation, or approximately 1 to 500 mg/adult/day of an injection is administered. However, the aforementioned numerical values are only given as examples. Thus, the dose is appropriately increased or decreased depending on various conditions such as the condition of a patient.

Next, the present invention will be specifically described in the following production examples, pharmaceutical preparation examples, and test examples of the propanedioic acid derivatives of the present invention. However, these examples are not intended to limit the scope of the present invention.

### Production Example 1

### Production of [3-[[6-(2-benzoxazolyl)-2-naphthalenyl]oxy]propyl]propanedioic acid:

6-(2-benzoxazolyl)-2-naphthalenol (372 mg) was dissolved in DMF (15 mL), and thereafter, potassium carbonate (1.0 g) and diethyl (3-chloropropyl)malonate (350 mg) were then added to the solution. The mixture was reacted at approximately 60°C overnight.
Thereafter, ethyl acetate (300 mL) was added to the reaction solution, and the mixture was then successively washed with water, and with a saturated saline solution. The ethyl acetate layer was dried with magnesium sulfate, followed by vacuum concentration. The residue was purified with a silica gel column (chloroform) to obtain [3-[[6-(2-benzoxazolyl)-2-naphthalenyl]oxy]propyl]propanedioic acid diethyl ester (299 mg, Y = 52%).
Thereafter, methanol (50 mL) and a 30% sodium hydroxide aqueous solution (1 mL) were added to the [3-[[6-(2-benzoxazolyl)-2-naphthalenyl]oxy]propyl]propanedioic acid diethyl ester (299 mg). The mixture was reacted at a room temperature overnight. The reaction solution was concentrated under a reduced pressure, and water (50 mL) was then added to the residue so as to dissolve it. Thereafter, diluted hydrochloric acid was added for precipitation. The precipitated crystal was collected by filtration, and it was then dried to obtain a compound of interest (174 mg, Y = 66%).

### Production Example 2

### Production of [3-[[6-[4-[(2-fluorophenyl)methoxy]-6-phenyl-2-pyrimidinyl]-2-naphthalenyl]oxy]propyl]propanedioic acid:

Potassium carbonate (276 mg) and diethyl (3-chloropropyl)malonate (284 mg) were added to 6-[4-[(2-fluorophenyl)methoxy]-6-phenyl-2-pyrimidinyl]-2-naphthalenol (422 mg) in DMF (10 ml). The obtained mixture was stirred at 80°C for 20 hours. The reaction solution was filtrated, and the filtrate was then concentrated under a reduced pressure. The residue was purified by a silica gel column (toluene-chloroform) to obtain [3-[[6-[4-[(2-fluorophenyl)methoxy]-6-phenyl-2-pyrimidinyl]-2-naphthalenyl]oxy]propyl]propanedioic acid diethyl ester in the form of an oily product. Thereafter, 1 N sodium hydroxide (2.5 ml) was added to the obtained ester in ethanol (10 ml), and the mixture was hydrolyzed under reflux for 1 hour. Thereafter, the solution was cooled, and the precipitate was then collected by filtration. The obtained crystal was dissolved in water (50 ml), by warming them, and the obtained solution was then filtrated. Thereafter, the filtrate was freeze-dried to obtain [3-[[6-[4-[(2-fluorophenyl)methoxy]-6-phenyl-2-pyrimidinyl]-2-naphthalenyl]oxy]propyl]propanedioic acid·2Na (171 mg) in the form of a white solid compound. The obtained crystal was dissolved in water, and it was then precipitated with 0.1 N hydrochloric acid, so as to obtain a compound of interest in the form of a white crystal.

The propanedioic acid derivatives of the present invention obtained by the same method as those described in the aforementioned production examples are shown in Tables 1 to 11.

**[Table 1]**

| Compound No. | Compound | Melting point (°C) | ¹H-NMR δ : |
|---|---|---|---|
| 1 | 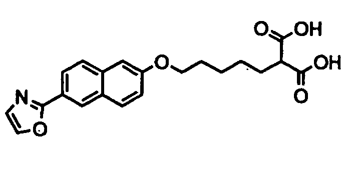 | | (DMSO-d6/TMS) 1. 49-1. 71(8H, m) 3. 25(1H , t, J=7Hz) 4. 12(2H, t, J=6 Hz) 7. 15-7. 42(3H, m) 7. 83-8. 24 (4H, m) 8. 49(1H, s ) 10. 94-13. 97(2H, br) |
| 2 | 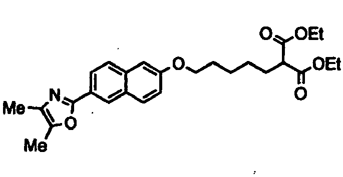 | 109 | (Chloroform-d/TMS) 1.26(6H,t,J=7Hz) 1.40-2 .05(8H,m) 2. 19(3H, s) 2 . 34(3H, s) 3. 35(1H, t, J=7 Hz) 3. 90-4. 38(6H, m) 7. 00-8. 14(5H, m) 8. 38 (1H, s) |
| 3 | 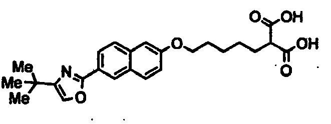 | | (DMSO-d6/TMS) 1. 30-2. 03(17H, m) 3. 24(1 H, t, J=7Hz) 4. 11(2H, t, J= 6Hz) 7. 21-7. 36 (3H, m) 7 . 81-8. 09 (3H, m) 8. 45(1H, s) 11. 82-13. 73 ( 2H, br) |
| 4 | 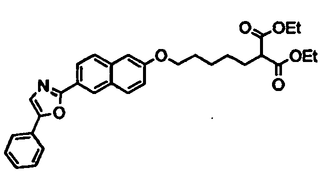 | 79 | (Chloroform-d/TMS) 1.26(6H, t, J=7Hz) 1.43-2 . 10 (8H, m) 3. 35 (1H, t, J=7 Hz) 4. 00-4. 39 (6H, m) 7. 10-8.24(11H, m) 8. 52 (1H, s) |

**[Table 2]**

| | | | |
|---|---|---|---|
| 5 | 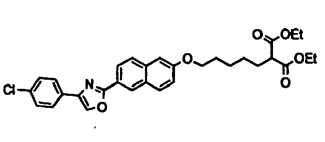 | 142 | (Chloroform-d/TMS) 1. 26 (6H, t, J=7Hz) 1.45-2.09(8H,m) 3. 35(1H, t, J =7Hz) 4. 00-4. 31 (6H, m) 7. 10-8. 24 (10H, m) 8. 50(1H, s) |
| 6 | 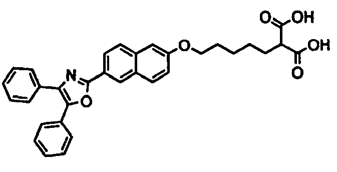 | | (DMSO-d6/TMS) 1.40-1.86(8H,m) 3. 17(1 H, t, J=7Hz) 4. 12(2H, t, J =6Hz) 7. 19-8. 25 (15H, m) 8. 62(1H, s) |
| 7 | 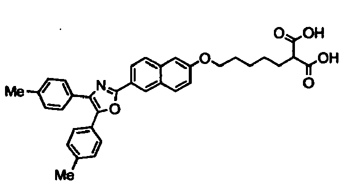 | | (DMSO-d6/TMS) 1. 43-1. 72 (8H, m) 2. 37 (6H, s) 3. 20(1H, t, J=7Hz) 4.12( 2H, t, J=6Hz) 7.21-8.0 7 (13H, m) 8. 58(1H, s) |
| 8 | 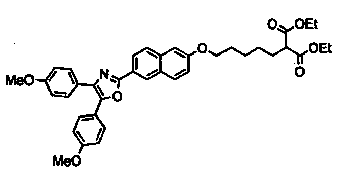 | | (Chloroform-d/TMS) 1. 14-2. 19(14H, m) 3. 35(1H, t, J=7Hz) 3. 86 (6H, s) 4. 00-4. 38 (6H, m) 6. 8 0-8.21(13H,m) 8. 53 (1H, s) |
| 9 | 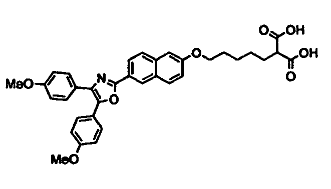 | | (DMSO-d6/TMS) 1. 48-1. 93 (8H, m) 3. 22(1 H, t, J=7Hz) 3. 83 (6H, s) 4. 12(2H, t, J=6Hz) 6. 9 8-8. 21(13H, m) 8. 57(1H, s) |

**[Table 3]**

| | | | |
|---|---|---|---|
| 10 | 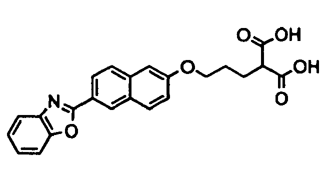 | 174 | (DMSO-d6/TMS) 1. 66-2. 09 (4H, m) 3. 38(1 H, t, J=6Hz) 4. 10-4. 30(2 H,m) 7. 23-8. 7 5(10H, m) 12. 82 (2H, br) |
| 11 | 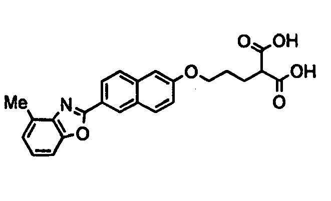 | | (DMSO-d6/TMS) 1. 69-2. 19 (4H, m) 2.62(3H,s) 3. 37(1H, t, J=7Hz) 4. 18 (2H, t, J=6Hz) 7. 07-8. 33 (8H, m) 8. 74(1H, s) 12 . 12-13. 22 (2H, br) |
| 12 | 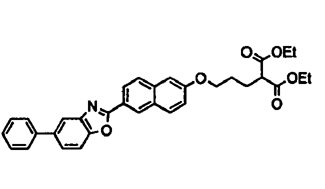 | | (Chloroform-d/TMS) 1. 28 (6H, t, J=7Hz) 1. 72-2. 34 (4H, m) 3. 47(1H, t, J =7Hz) 3.97-4. 40(6H,m) 7. 15-8. 38 (13 H, m) 8. 71(1H, s) |
| 13 | 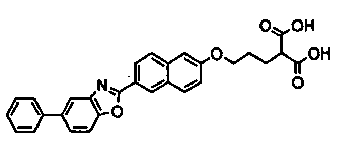 | | (DMSO-d6/TMS) 1.89-1.99(4H,m) 3. 38(1 H,t,J=4Hz) 4.19(2H,t,J =5Hz) 7. 23-8. 3 4(13H, m) 8. 77(1H, s) 12. 28-13. 19 (2H, br) |
| 14 | 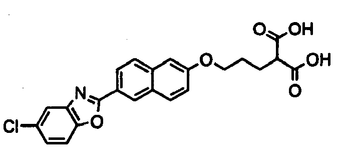 | 171 | (DMSO-d6/TMS) 1. 75-2. 10 (4H, m) 3. 37(1 H, t, J=6Hz) 4. 17(2H, t, J =7Hz) 7.21-8. 72 (9H, m) 12. 75 (2H, br) |

**[Table 4]**

| | | | |
|---|---|---|---|
| 15 | 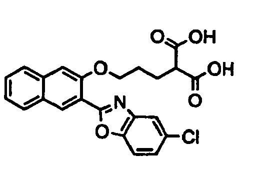 | 161 (Decomposed) | (DMSO-d6/TMS) 1. 73-2. 29(4H, m) 3. 45(1 H, t, J=7Hz) 4. 28(2H, t, J =6Hz) 7. 42-8. 14 (8H, m) 8. 73 (1H, s) 12. 68 (2H, br) |
| 16 | 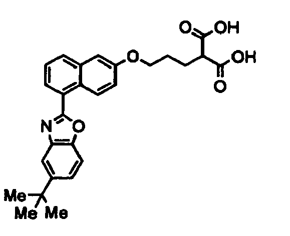 | | (DMSO-d6/TMS) 1. 40 (9H, s) 1. 74-2. 14 (4H, m) 3. 37 (1 H, t, J=6Hz) 4 . 19 (2H, t, J=5Hz) 7. 31-8 . 34 (8H, m) 9. 37(1H, d, J= 9Hz) |
| 17 | 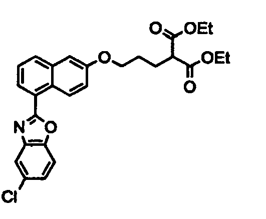 | 96 | (Chloroform-d/TMS) 1. 27 (6H, t, J=7Hz) 1. 79-2. 28 (4H, m) 3. 47(1H, t, J =7Hz) 4.05-4. 40 (6H, m) 7. 18-8. 35(8H, m) 9. 36(1H, d, J=9Hz) |
| 18 | 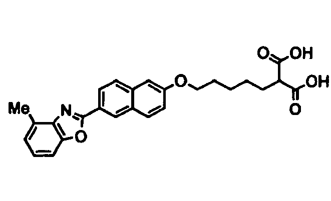 | | (DMSO-d6/TMS) 1. 05-2. 17 (8H, m) 2. 62(3H, s) 3. 26(1H, t, J=6Hz) 4. 13(2H, t, J=6Hz) 7 . 24-8. 36 (8H, m) 8. 73 (1H, s) 1 1. 91-13. 24 (2H, br) |

**[Table 5]**

| | | | |
|---|---|---|---|
| 19 | 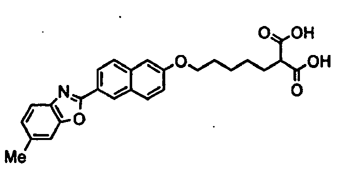 | | (DMSO-d6/TMS) 1. 18-1. 99 (8H, m) 2. 49 (3H, s) 3. 25(1H, t, J=7Hz) 4. 12(2H, t, J=6Hz) 7. 17-8. 29 (8H, m) 8. 70(1H, s) 12 . 15-13. 19 (2H, br) |
| 20 | 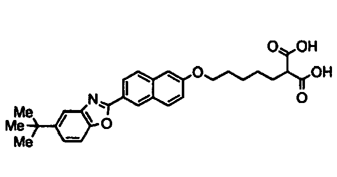 | | (DMSO-d6/TMS) 1. 38-1. 98 (17H, m) 3. 25( 1H, t, J=7Hz) 4.14(2H,t, J=5Hz) 7.21-8 . 30 (8H, m) 8. 72(1H, s) 11. 48-14. 04 (2H, br) |
| 21 | 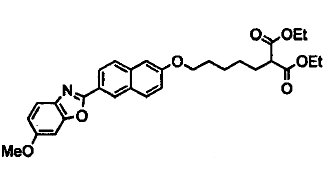 | | (Chloroform-d/TMS) 1. 08-2. 20 (14H, m) 3. 36( 1H, t, J=7Hz) 3. 74-4. 55 ( 9H,m) 6.87-8.62 (9H, m) |
| 22 | 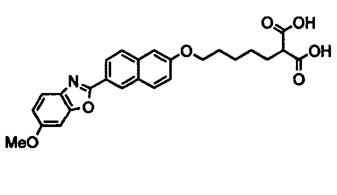 | 175 | (DMSO-d6/TMS) 1. 20-1. 94 (8H, m) 3. 25( 1H, t, J=7Hz) 3. 87(3H, s) 4. 13(2H, t, J=6Hz) 6.95-8. 64 (9H, m) |

**[Table 6]**

| | | | |
|---|---|---|---|
| 23 | 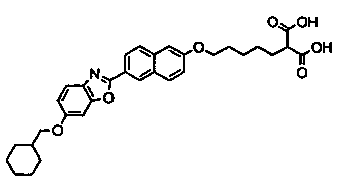 | | (DMSO-d6/TMS) 1. 00-2. 02 (19H, m) 3. 05-4. 30 (5H, m) 6.90-8.64(9 H, m) 12. 6(2H , br) |
| 24 | 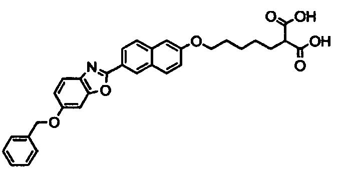 | | (DMSO-d6/TMS) 1. 25-2. 0 (8H, m) 3. 26(1H, t, J=7Hz) 4. 18(2H, t, J=7Hz) 5. 22 (2H, s) 7. 1 8-8. 65(14H, m) |
| 25 | 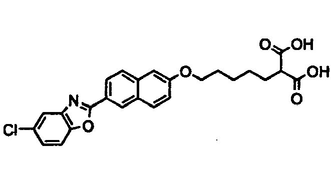 | 184 | (DMSO-d6/TMS) 1. 21-1. 95(8H, m) 3. 25(1 H, t, J=7Hz) 4. 12(2H, t, J =6Hz) 7.20-8. 71 (9H, m) 12. 7(2H, br) |
| 26 | 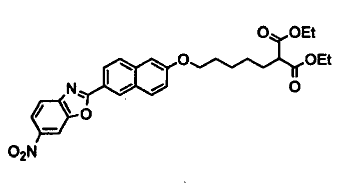 | 120 | (Chloroform-d/TMS) 1. 15-2. 20 (14H, m) 3. 36( 1H, t, J=7Hz) 4. 04-4. 44 ( 6H, m) 7. 14-8. 70 (9H, m) |
| 27 | 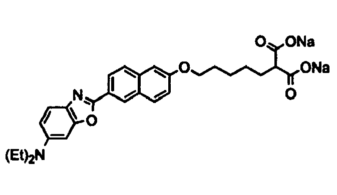 | | (Methanol-d4, D₂0/TMS) 1. 01-1. 99(14H, m) 3.00-3. 59 (5H, m) 4. 11(2H, t, J =5Hz) 6.90-8.42 (9H, m) |

**[Table 7]**

| | | | |
|---|---|---|---|
| 28 | 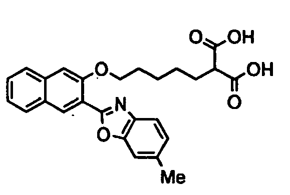 | | (DMSO-d6/TMS) 1. 19-1. 78(8H, m) 2. 51 (3H, s) 3. 22(1H, t, J=6Hz) 4. 23(2H, t, J=5Hz) 7. 19-8. 10(8H, m) 8. 63(1H, s) |
| 29 | 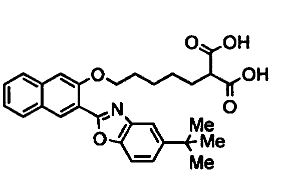 | | (DMSO-d6/TMS) 1. 39-2. 09(17H, m) 3. 25( 1H, t, J=7Hz) 4. 23(2H, t, J=5Hz) 7.40-8. 11 (8H, m) 8. 63(1H, s) 12.02-13.40 (2H, br) |
| 30 | 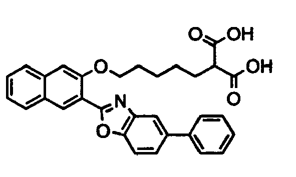 | | (DMSO-d6/TMS) 1. 18-2. 08 (8H, m) 3. 16(1 H, t, J=6Hz) 4. 25(2H, t, J =5Hz) 7. 42-8. 1 0 (13H, m) 8. 70(1H, s) |
| 31 | 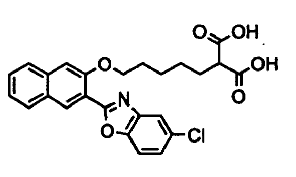 | 100 | (DMSO-D6/TMS) 1. 29-2. 10 (8H, m) 3. 19(1 H, t. J=3Hz) 4. 23(2H, t, J =5Hz) 7.38-8. 11 (8H, m) 8. 68 (1H, s) |
| 32 | 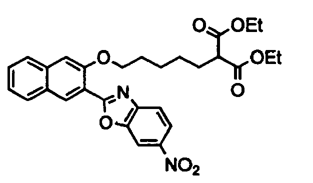 | 95 | (Chloroform-d/TMS) 1. 13-2. 20 (14H, m) 3. 35( 1H, t, J=7Hz) 4. 02-4. 37 ( 6H,m) 7.42-8.72 (9H, m) |

**[Table 8]**

| | | | |
|---|---|---|---|
| 33 | 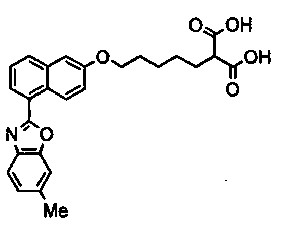 | | (DMSO-d6/TMS) 1. 15-1. 80 (8H, m) 2. 51 (3H, s) 3. 25(1H, t, J=6Hz) 4. 13 (2H, t, J=5Hz) 7.21-8.30(8H,m) 9.36 (1H, d, J=9Hz) 11. 48-13. 83(2H, br) |
| 34 | 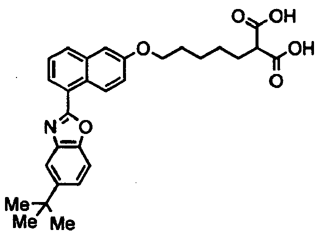 | | (DMSO-d6/TMS) 1. 40-2. 08(17H, m) 3. 24( 1H, t, J=7Hz) 4.15(2H,t, J=5Hz) 7.21-8. 32(8H,m) 9. 37(1H, d, J=9 Hz) |
| 35 | 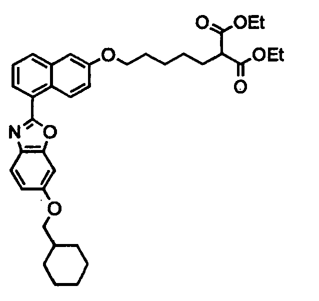 | 107 | (Chloroform-d/TMS) 1.14-2.15(25H,m) 3. 35( 1H, t, J=7Hz) 3. 79-4. 39 ( 8H, m) 6. 88-8. 29(8H,m) 9. 37(1H,d,J=9 Hz) |
| 36 | 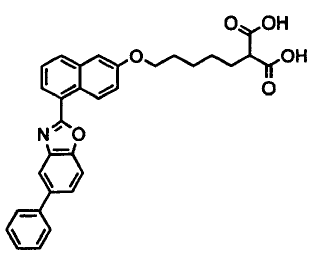 | | (DMSO-d6/TMS) 1. 43-1. 83 (8H, m) 3. 25(1 H,t,J=6Hz) 4.14(2H,t,J =4Hz) 7.21-8 . 36 (13H, m) 9. 40(1H, d, J =10Hz) 12. 65(2H, brs) |

**[Table 9]**

| | | | |
|---|---|---|---|
| 37 | 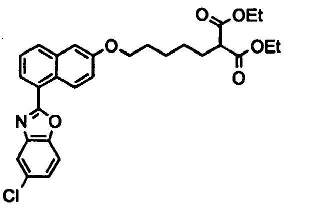 | 73 | (Chloroform-d/TMS) 1. 15-2. 20(14H, m) 3. 36( 1H, t, J=7Hz) 4. 03-4. 43( 6H,m) 7.18-8. 34(8H,m) 8. 85(1H, d, J=9 Hz) |
| 38 | 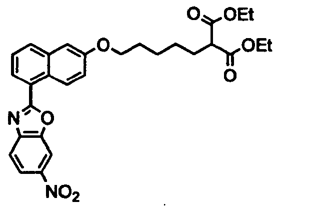 | 105 | (Chloroform-d/TMS) 1. 15-2. 18(14H, m) 3. 36( 1H, t, J=6Hz) 4. 03-4. 40 ( 6H, m) 7. 23-8. 52(8H,m) 9. 39(1H, d, J=9 Hz) |
| 39 | 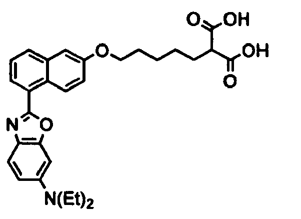 | | (DMSO-d6/TMS) 1.03-2. 41(14H, m) 3. 14-3. 65(5H, m) 4. 14(2H, t, J =5Hz) 6.79-8.25 (8H,m) 9. 40(1H, d, J=9Hz ) 12. 5(2H, br) |
| 40 | 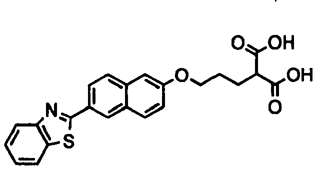 | 174 | (DMSO-d6/TMS) 1. 70-2. 00 (4H, m) 3. 38(1 H, t, J=6Hz) 4. 18(2H, t, J =5Hz) 7.18-8.6 1(10H, m) 12.7(2H,br) |
| 41 | 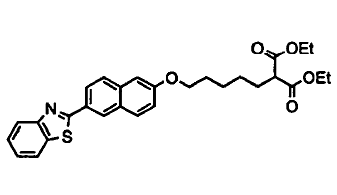 | 109 | (Chloroform-d/TMS) 1. 14-2. 15 (14H, m) 3. 35( 1H, t, J=7Hz) 4. 02-4. 39( 6H,m) 7. 13-8. 4 8(10H, m) |

**[Table 10]**

| | | | |
|---|---|---|---|
| 42 | 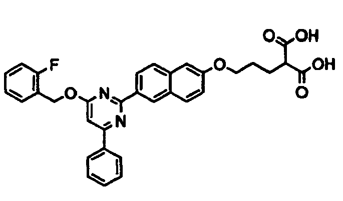 | | (DMSO-d6/TMS) 1. 75-2. 15 (4H, m) 3. 36(1H , t, J=6Hz) 4. 19 (2H, t, J=6 Hz) 5. 75 (2H, s) 7. 09-8.70(15H,m) 9. 04(1H, s) |
| 43 | 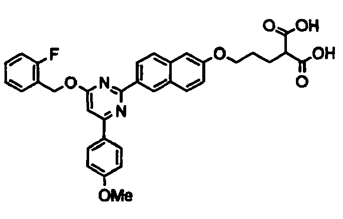 | 1 0 3 (Decomposed) | (DMSO-d6/TMS) 1. 89-1. 90 (4H, m) 3. 37(1H , t, J=6Hz) 3. 87(3H, s) 4. 17 (2H, t, J=5Hz) 5. 73 (2H, s) 7. 05-8. 70 (14H, m) 9. 03 (1H, s) |
| 44 | 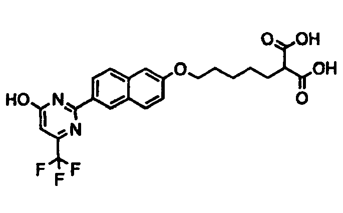 | | (DMSO-d6/TMS) 1. 48-2. 10 (8H, m) 3. 23(1H , t, J=6Hz) 4. 14 (2H, t, J=6 Hz) 6. 85(1H, s) 7. 20-7. 50 (2H, m) 7. 86-8. 26 (3H,m) 8. 72(1H, s) 12. 0 2-13. 46 (2H, br) |
| 45 | 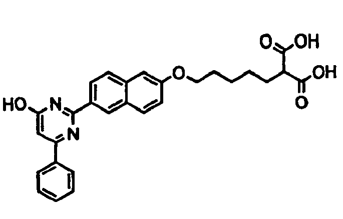 | | (DMSO-d6/TMS) 1. 23-1. 98 (8H, m) 3. 24(1H , t, J=6Hz) 4. 15 (2H, t, J=5 Hz) 6. 91 (1H, s) 7. 21-7. 59(5H, m) 7.91-8.44 (5H, m) 8. 82(1H, s) 12. 2 3-13. 25(2H, br) |

**[Table 11]**

| | | | |
|---|---|---|---|
| 46 | 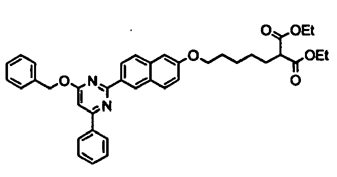 | | (Chloroform-d/TMS) 1. 14-2. 20(14H, m) 3.36(1 H, t, J=7Hz) 4. 00-4. 39 (6H , m) 5. 68(2H, s) 7 . 00-8. 30(15H, m) 8. 56-8. 74 (1H, dd) 9. 01(1H, s) |
| 47 | 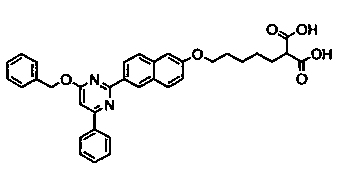 | | (DMSO-d6/TMS) 1. 22-1. 97 (8H, m) 3. 21(1H , t, J=6Hz) 4. 13(2H, t, J=6 Hz) 5. 70 (2H, s) 7. 14-7. 62(11H, m) 7. 80-8. 1 7(2H, m) 8. 31-8. 71 (3H, m) 9. 02(1H, s) |
| 48 | 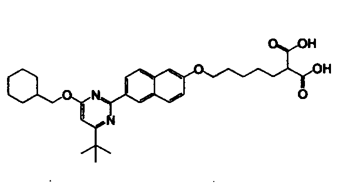 | | 0. 82-1. 92 (28H, m) 3. 23(1H, t, J=6Hz) 4. 03-4. 39 (4H, m) 6. 74(1H, s) 7. 14-7. 35 (2H, m) 7. 82-8. 08(2H, m) 8. 48(1H, d, J=9Hz) 8. 87(1H, s) |
| 49 | 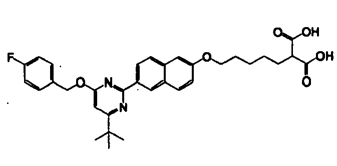 | | 1. 18-1. 92 (17H, m) 3. 21 (1H, t, J=6Hz) 4. 13(2H, t, J=6Hz) 5. 61 (2H, s) 6. 82(1H, s) 7. 10-8. 10 (8H, m) 8. 52 (1H, d, J=9Hz) 8. 91(1H, s) |
| 50 | 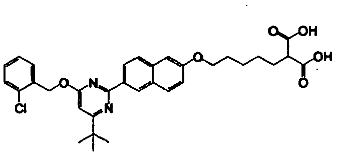 | | 1. 23-2. 03(17H, m) 3. 21 (1H, t, J=7Hz) 4. 13 (2H, t, J=6Hz) 5. 71(2H, s) 6. 86 (1H, s) 7. 14-8. 06(8H, m) 8. 50 (1H, d, J=8Hz) 8. 90(1H, s) |

### Pharmaceutical Preparation Example 1

**Tablet:**

| | |
|---|---|
| Propanedioic acid derivative of the present invention | 10.0 g |
| Lactose | 9.0 g |
| Hydroxypropylcellulose | 2.0 g |
| Crystalline cellulose | 7.7 g |
| Magnesium stearate | 0.3 g |
| Talc | 1.0 g |

Using the aforementioned components, a tablet that contains 100 mg of the propanedioic acid derivative of the present invention was produced according to an ordinary method.

### Test Example 1

### PAI-1 inhibitory activity:

PAI-1 inhibitory activity can be measured using a synthetic substrate S-2288 according to the methods described in International Publication WO04/011442 pamphlet, International Publication WO04/10996 pamphlet, JP-A-2004-250401, etc.
Representative examples of such PAI-1 inhibitory activity value are shown in Table 12.

**[Table 12]**

| Compound No. | IC50 (M) |
|---|---|
| 6 | 9.4 × 10-7 |
| 7 | 5.9 × 10-7 |
| 9 | 3.6 × 10-7 |
| 13 | 5.4 × 10-7 |
| 20 | 4.4 × 10-7 |
| 23 | 3.9 × 10-7 |
| 24 | 5.4 × 10-7 |
| 43 | 8.6 × 10-7 |
| 47 | 3.5 × 10-7 |
| 48 | 3.2 × 10-7 |
| 49 | 2.9 × 10-7 |
| 50 | 2.2 × 10-7 |

### Test Example 2

### Cancer suppression test:

### (1) Test method

5-week-old male Min mice (CLEA Japan, Inc.) were purchased. After giving a basic feed (AIN-76A) to the mice for 1 week, 100 ppm of a test substance was mixed into the feed, and the mixed feed was then given to the mice. On the other hand, in the case of wild-type mice also, the same amount of the test substance was mixed into the feed, and the mixed feed was then given to the wild-type mice. The body weight and the feed intake were measured every week. The mice were sacrificed under anesthesia, when they were 15 weeks old. Then, the weights of main organs (spleen and heart) were measured. Small intestine and large intestine were washed and were then excised in a longitudinal direction, followed by fixing with formalin. The number of intestinal polyps in the small intestine and large intestine were counted and the size thereof were measured under a stereoscopic microscope.

### (2) Results

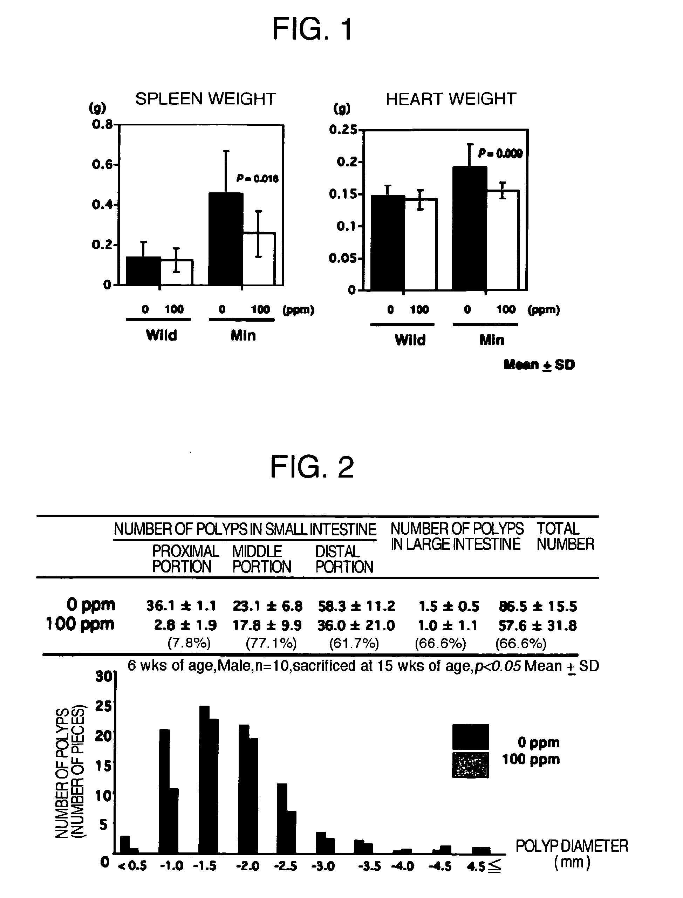

The results of the test using Compound No. 20 are shown in Figs. 1 and 2. From Figs. 1 and 2, the following points were discovered.
1. Evaluation based on the measurement of the weights of spleen and heart used as indicators of anemia caused by bleeding associated with the development of intestinal polyps: In Compound No. 20 administration group, the weights of spleen and heart were significantly reduced.
2. The number of intestinal polyps per mouse: The development of intestinal polyps was suppressed particularly strongly in the proximal portion of small intestine. The total number of polyps was decreased to approximately 66%.
3. With regard to distribution of the polyp size, a decrease in the size was observed overall. In particular, it was observed that polyps with a diameter size of 1 mm or less were significantly decreased.
From the aforementioned test results, it was confirmed that the propanedioic acid derivative of the present invention, a compound having PAI-1 inhibitory activity, is effective for suppressing the development of gastrointestinal polyp and malignant tumor and the progression thereof.

### INDUSTRIAL APPLICABILITY

The propanedioic acid derivative of the present invention can be used as an agent for preventing or treating gastrointestinal polyp, gastrointestinal cancer (esophageal cancer, stomach cancer, duodenal cancer, small intestinal cancer, large intestinal cancer (e.g. colon cancer, rectal cancer)), lung cancer, breast cancer, pancreatic cancer, liver cancer, uterine cancer, ovarian cancer, epithelial malignant tumor, prostatic cancer, leukemia, malignant lymphoma, multiple myeloma, and the like, and/or as an agent for preventing the metastasis of such malignant tumor. In particular, the propanedioic acid derivative of the present invention is useful as an agent for preventing or treating gastrointestinal polyp and gastrointestinal cancer (esophageal cancer, stomach cancer, duodenal cancer, small intestinal cancer, large intestinal cancer (colon cancer, rectal cancer)), and/or as an agent for preventing the metastasis of such malignant tumor.

### BRIEF DESCRIPTION OF THE DRAWINGS

Fig. 1 shows evaluation of the effect of the propanedioic acid derivative of the present invention on the weights of spleen and heart that are used as indicators of anemia caused by bleeding associated with the development of intestinal polyps.
Fig. 2 shows evaluation of the effect of the propanedioic acid derivative of the present invention on the number of intestinal polyps found in small intestine and large intestine.

## Claims

1. An agent for preventing or treating gastrointestinal polyp and/or malignant tumor, or an agent for preventing the metastasis of malignant tumor, which comprises a propanedioic acid derivative represented by the following formula (1), a nontoxic salt thereof, a solvate thereof, or a prodrug thereof, as an active ingredient, 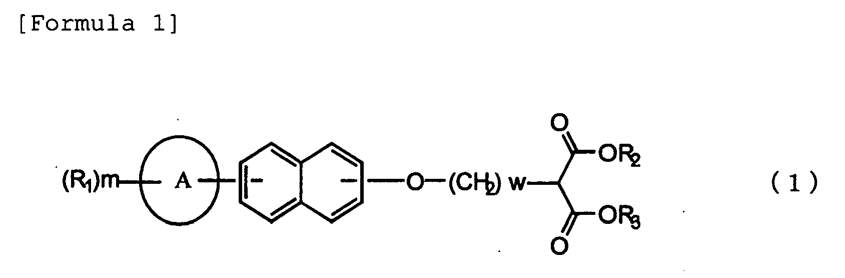 wherein ring A represents oxazole, benzoxazole, benzothiazole, or pyrimidine; R₁ represents a hydroxyl group, an alkyl group, a lower alkoxy group, a cyclohexylmethoxy group, a benzyloxy group (wherein the phenyl of the benzyloxy group may be substituted with a substituent selected from among a lower alkyl group, a lower alkoxy group, and a halogen atom), a trifluoromethyl group, a nitro group, a -N(R₄,R₅) group (wherein each of R₄ and R₅ independently represents a hydrogen atom, an alkyl group, or a benzyl group), a halogen atom, or a phenyl group (wherein the phenyl group may be substituted with a substituent selected from among a lower alkyl group, a lower alkoxy group, and a halogen atom); each of R₂ and R₃ independently represents a hydrogen atom or a lower alkyl group; m represents 0 or an integer of 1 to 4, and an m number of R₁ may be identical to or different from one another; and W represents an integer of 3 to 5.

2. The agent for preventing or treating gastrointestinal polyp and/or malignant tumor, or the agent for preventing the metastasis of malignant tumor according to claim 1, which comprises a propanedioic acid derivative represented by the following formula (2), a nontoxic salt thereof, a solvate thereof, or a prodrug thereof, as an active ingredient, 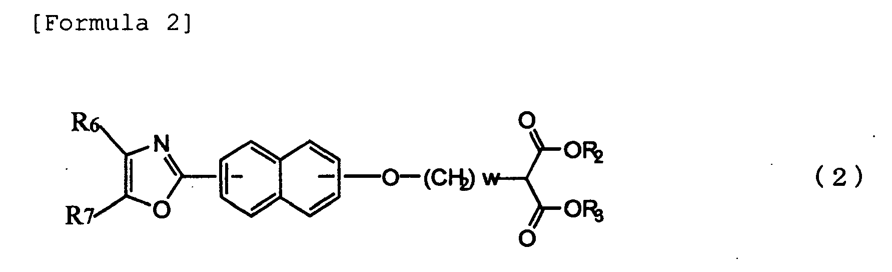 wherein R₂, R₃, and W have the same meanings as those described above, and R₆ and R₇ identically or differently represent a hydrogen atom, an alkyl group, or a phenyl group (wherein the phenyl group may be substituted with a substituent selected from among a lower alkyl group, a lower alkoxy group, and a halogen atom).

3. The agent for preventing or treating gastrointestinal polyp and/or malignant tumor, or the agent for preventing the metastasis of malignant tumor according to claim 1, which comprises a propanedioic acid derivative represented by the following formula (3), a nontoxic salt thereof, a solvate thereof, or a prodrug thereof, as an active ingredient, 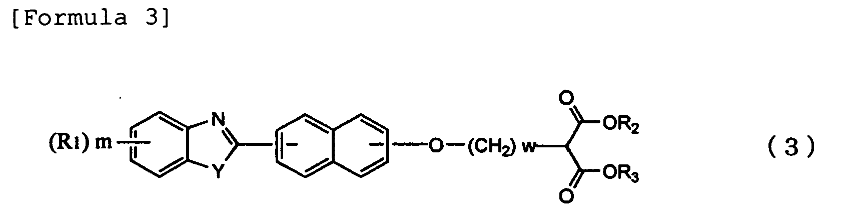 wherein Y represents an oxygen atom or a sulfur atom, and R₁, R₂, R₃, m, and W have the same meanings as those described above.

4. The agent for preventing or treating gastrointestinal polyp and/or malignant tumor, or the agent for preventing the metastasis of malignant tumor according to claim 3, which comprises a propanedioic acid derivative represented by the following formula (4), a nontoxic salt thereof, a solvate thereof, or a prodrug thereof, as an active ingredient, 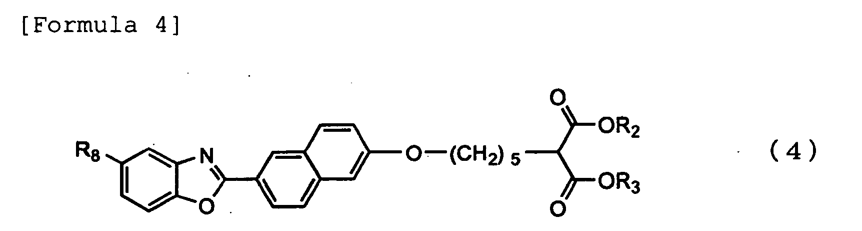 wherein R₂ and R₃ have the same meanings as those described above, and R₈ represents an alkyl group.

5. The agent for preventing or treating gastrointestinal polyp and/or malignant tumor, or the agent for preventing the metastasis of malignant tumor according to claim 4, which comprises a propanedioic acid derivative represented by the following formula (5), a nontoxic salt thereof, a solvate thereof, or a prodrug thereof, as an active ingredient, 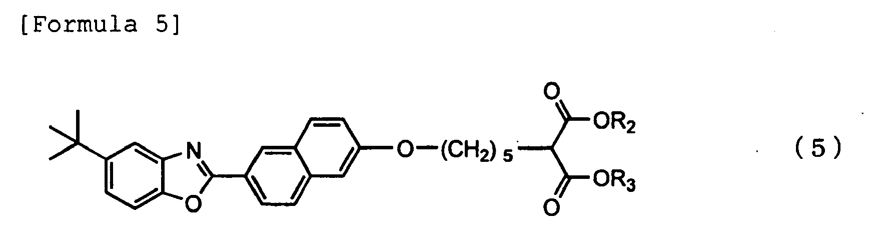 wherein R₂ and R₃ have the same meanings as those described above.

6. The agent for preventing or treating gastrointestinal polyp and/or malignant tumor, or the agent for preventing the metastasis of malignant tumor according to claim 1, which comprises a propanedioic acid derivative represented by the following formula (6), a nontoxic salt thereof, a solvate thereof, or a prodrug thereof, as an active ingredient, 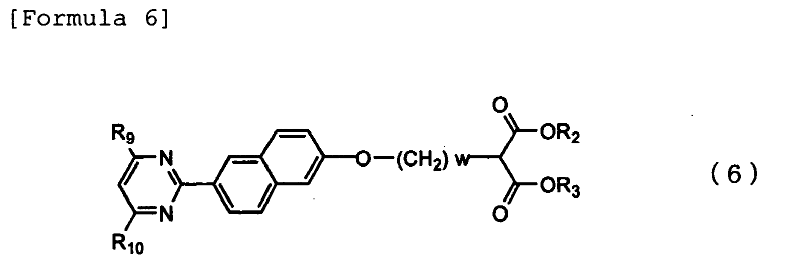 wherein R₂, R₃, and W have the same meanings as those described above; R₉ represents a hydroxyl group, a cyclohexylmethoxy group, or a benzyloxy group (wherein the phenyl of the benzyloxy group may be substituted with a substituent selected from among a lower alkyl group, a lower alkoxy group, and a halogen atom); and R₁₀ represents an alkyl group, a trifluoromethyl group, or a phenyl group (wherein the phenyl group may be substituted with a substituent selected from among a lower alkyl group, a lower alkoxy group, and a halogen atom).

7. The agent for preventing or treating gastrointestinal polyp and/or malignant tumor, or the agent for preventing the metastasis of malignant tumor according to any one of claims 1 to 6, which comprises a propanedioic acid derivative selected from the group consisting of the following compounds, a nontoxic salt thereof, a solvate thereof, or a prodrug thereof, as an active ingredient,
[5-[[6-(2-oxazolyl)-2-naphthalenyl]oxy]pentyl]propanedioic acid;
[5-[[6-(4,5-dimethyl-2-oxazolyl)-2-naphthalenyl]oxy]pentyl]propanedioic acid diethyl ester;
[5-[[6-[4-(1,1-dimethylethyl)-2-oxazolyl]-2-naphthalenyl]oxy]pentyl]propanedioic acid;
[5-[[6-(5-phenyl-2-oxazolyl)-2-naphthalenyl]oxy]pentyl]propanedioic acid diethyl ester;
[5-[[6-[4-(4-chlorophenyl)-2-oxazolyl]-2-naphthalenyl]oxy]pentyl]propanedioic acid diethyl ester;
[5-[[6-(4,5-diphenyl-2-oxazolyl)-2-naphthalenyl]oxy]pentyl]propanedioic acid;
[5-[[6-[4,5-bis(4-methylphenyl)-2-oxazolyl]-2-naphthalenyl]oxy]pentyl]propanedioic acid;
[5-[[6-[4,5-bis(4-methoxyphenyl)-2-oxazolyl]-2-naphthalenyl]oxy]pentyl]propanedioic acid diethyl ester;
[5-[[6-[4,5-bis(4-methoxyphenyl)-2-oxazolyl]-2-naphthalenyl]oxy]pentyl]propanedioic acid;
[3-[[6-(2-benzoxazolyl)-2-naphthalenyl]oxy]propyl]propanedioic acid;
[3-[[6-(4-methyl-2-benzoxazolyl)-2-naphthalenyl]oxy]propyl]propanedioic acid;
[3-[[6-(5-phenyl-2-benzoxazolyl)-2-naphthalenyl]oxy]propyl]propanedioic acid diethyl ester;
[3-[[6-(5-phenyl-2-benzoxazolyl)-2-naphthalenyl]oxy]propyl]propanedioic acid;
[3-[[6-(5-chloro-2-benzoxazolyl)-2-naphthalenyl]oxy]propyl]propanedioic acid;
[3-[[3-(5-chloro-2-benzoxazolyl)-2-naphthalenyl]oxy]propyl]propanedioic acid;
[3-[[5-[5-(1,1-dimethylethyl)-2-benzoxazolyl]-2-naphthalenyl]oxy]propyl]propanedioic acid;
[3-[[5-(5-chloro-2-benzoxazolyl)-2-naphthalenyl]oxy]propyl]propanedioic acid diethyl ester;
[5-[[6-(4-methyl-2-benzoxazolyl)-2-naphthalenyl]oxy]pentyl]propanedioic acid;
[5-[[6-(6-methyl-2-benzoxazolyl)-2-naphthalenyl]oxy]pentyl]propanedioic acid;
[5-[[6-[5-(1,1-dimethylethyl)-2-benzoxazolyl]-2-naphthalenyl]oxy]pentyl]propanedioic acid;
[5-[[6-(6-methoxy-2-benzoxazolyl)-2-naphthalenyl]oxy]pentyl]propanedioic acid diethyl ester;
[5-[[6-(6-methoxy-2-benzoxazolyl)-2-naphthalenyl]oxy]pentyl]propanedioic acid;
[5-[[6-[6-(cyclohexylmethoxy)-2-benzoxazolyl]-2-naphthalenyl]oxy]pentyl]propanedioic acid;
[5-[[6-[6-(phenylmethoxy)-2-benzoxazolyl]-2-naphthalenyl]oxy]pentyl]propanedioic acid;
[5-[[6-(5-chloro-2-benzoxazolyl)-2-naphthalenyl]oxy]pentyl]propanedioic acid;
[5-[[6-(6-nitro-2-benzoxazolyl)-2-naphthalenyl]oxy]pentyl]propanedioic acid diethyl ester;
[5-[[6-[6-(diethylamino)-2-benzoxazolyl]-2-naphthalenyl]oxy]pentyl]propanedioic acid disodium salt;
[5-[[3-(6-methyl-2-benzoxazolyl)-2-naphthalenyl]oxy]pentyl]propanedioic acid;
[5-[[3-[5-(1,1-dimethylethyl)-2-benzoxazolyl]-2-naphthalenyl]oxy]pentyl]propanedioic acid;
[5-[[3-(5-phenyl-2-benzoxazolyl)-2-naphthalenyl]oxy]pentyl]propanedioic acid;
[5-[[3-(5-chloro-2-benzoxazolyl)-2-naphthalenyl]oxy]pentyl]propanedioic acid;
[5-[[3-(6-nitro-2-benzoxazolyl)-2-naphthalenyl]oxy]pentyl]propanedioic acid diethyl ester;
[5-[[5-(6-methyl-2-benzoxazolyl)-2-naphthalenyl]oxy]pentyl]propanedioic acid;
[5-[[5-[5-(1,1-dimethylethyl)-2-benzoxazolyl]-2-naphthalenyl]oxy]pentyl]propanedioic acid;
[5-[[5-[6-(cyclohexylmethoxy)-2-benzoxazolyl]-2-naphthalenyl]oxy]pentyl]propanedioic acid diethyl ester;
[5-[[5-(5-phenyl-2-benzoxazolyl)-2-naphthalenyl]oxy]pentyl]propanedioic acid;
[5-[[5-(5-chloro-2-benzoxazolyl)-2-naphthalenyl]oxy]pentyl]propanedioic acid diethyl ester;
[5-[[5-(6-nitro-2-benzoxazolyl)-2-naphthalenyl]oxy]pentyl]propanedioic acid diethyl ester;
[5-[[5-[6-(diethylamino)-2-benzoxazolyl]-2-naphthalenyl]oxy]pentyl]propanedioic acid;
[3-[[6-(2-benzothiazolyl)-2-naphthalenyl]oxy]propyl]propanedioic acid;
[5-[[6-(2-benzothiazolyl)-2-naphthalenyl]oxy]pentyl]propanedioic acid diethyl ester;
[3-[[6-[4-[(2-fluorophenyl)methoxy]-6-phenyl-2-pyrimidinyl]-2-naphthalenyl]oxy]propyl]propanedioic acid;
[3-[[6-[4-[(2-fluorophenyl)methoxy]-6-(4-methoxyphenyl)-2-pyrimidinyl]-2-naphthalenyl]oxy]propyl]propanedioic acid;
[5-[[6-[1,4-dihydro-4-oxo-6-(trifluoromethyl)-2-pyrimidinyl]-2-naphthalenyl]oxy]pentyl]propanedioic acid;
[5-[[6-(1,4-dihydro-4-oxo-6-phenyl-2-pyrimidinyl)-2-naphthalenyl]oxy]pentyl]propanedioic acid;
[5-[[6-[4-phenyl-6-(phenylmethoxy)-2-pyrimidinyl]-2-naphthalenyl]oxy]pentyl]propanedioic acid ethyl ester;
[5-[[6-[4-phenyl-6-(phenylmethoxy)-2-pyrimidinyl]-2-naphthalenyl]oxy]pentyl]propanedioic acid;
[5-[[6-[4-cyclohexylmethoxy-6-(1,1-dimethylethyl)-2-pyrimidinyl]-2-naphthalenyl]oxy]pentyl]propanedioic acid;
[5-[[6-[4-(1,1-dimethylethyl)-6-[(4-fluorophenyl)methoxy]-2-pyrimidinyl]-2-naphthalenyl]oxy]pentyl]propanedioic acid; and
[5-[[6-[4-[(2-chlorophenyl)methoxy]-6-(1,1-dimethylethyl)-2-pyrimidinyl]-2-naphthalenyl]oxy]pentyl]propanedioic acid.

8. The agent for preventing or treating gastrointestinal polyp and/or malignant tumor, or the agent for preventing the metastasis of malignant tumor according to any one of claims 1 to 7, wherein the malignant tumor is lung cancer, breast cancer, pancreatic cancer, liver cancer, gastrointestinal cancer, uterine cancer, ovarian cancer, epithelial malignant tumor, prostatic cancer, leukemia, malignant lymphoma, or multiple myeloma.

9. The agent for preventing or treating gastrointestinal polyp and/or malignant tumor, or the agent for preventing the metastasis of malignant tumor according to claim 8, wherein the gastrointestinal cancer is esophageal cancer, stomach cancer, duodenal cancer, small intestinal cancer, or large intestinal cancer.

10. The agent for preventing or treating gastrointestinal polyp and/or malignant tumor, or the agent for preventing the metastasis of malignant tumor according to claim 9, wherein the large intestinal cancer is colon cancer or rectal cancer.

11. Use of the propanedioic acid derivative, a nontoxic salt thereof, a solvate thereof, or a prodrug thereof according to any one of claims 1 to 7 for production of the agent for preventing or treating gastrointestinal polyp and/or malignant tumor, or the agent for preventing the metastasis of malignant tumor.

12. A method for preventing or treating gastrointestinal polyp and/or malignant tumor, or preventing the metastasis of malignant tumor, which comprises administration of the propanedioic acid derivative, a nontoxic salt thereof, a solvate thereof, or a prodrug thereof according to any one of claims 1 to 7 to humans or animals.

13. The method according to claim 12, which prevents or treats gastrointestinal cancer.

14. The method according to claim 13, wherein the gastrointestinal cancer is esophageal cancer, stomach cancer, duodenal cancer, small intestinal cancer, or large intestinal cancer.
